# EUROPEAN PATENT SPECIFICATION

(11) **EP 3 860 671 B1**
(45) Date of publication and mention of the grant of the patent: **25.12.2024**
(21) Application number: 19783468.2
(22) Date of filing: 01.10.2019
(51) Int. Cl.: A61M 1/00

(54) **DRAINAGE DEVICE WITH PRESSURE RELIEF VALVE**
DRAINAGEVORRICHTUNG MIT DRUCKENTLASTUNGSVENTIL
DISPOSITIF DE DRAINAGE AVEC SOUPAPE DE SURPRESSION

(30) Priority: 02.10.2018 SE 1851183
(43) Date of publication of application: 11.08.2021
(73) Proprietor: Tintron AB, 411 36 Göteborg (SE)
(72) Inventor: CHARLÉZ, Mikael, 412 57 Göteborg (SE)
(74) Representative: Hansson Thyresson AB
(86) International application number: PCT/EP2019/076540
(87) International publication number: WO 2020/070103

(56) References cited:
- WO-A2-2017/152125
- US-A1- 2004 260 255

## Description

### TECHNICAL FIELD

The present invention relates to a drainage device for removing fluid from a body cavity of a patient, more precisely a drainage device with a pressure sensing means.

### BACKGROUND

In contemporary medical care, the movement of fluid from a body cavity to another point for collection is a routine need and can be performed in several ways. When tubing or piping is used for carrying the fluid during the movement either gravity or a pump is utilized to create and/or sustain a suction pressure needed to move the fluid from one point to another.

At times the movement of fluid from the body must be performed in a gentle, slow and steady manner. Such gentle, slow and steady manner can be designated "peristalsis". Peristaltic pumping may be performed in a number of ways including, but not exclusively, by hand pump or with the use of a peristaltic pump.

Additional known methods for drainage procedures include plastic vacuum suction bottles and wall/portable suction. These methods typically produce a constant suction rather than a peristaltic suction. These methods also include plastic bottles that are pre-assembled with a vacuumed pre-set under pressure causing inadequate suction.

Since these pumps are not monitored at all times by medical staff, a problem may occur when the excess fluid has been removed from the patient. The pump continues to run until it is being shut down, and during this time the pumping causes severe pain to the patient.

From the above it is understood that there is room for improvements and the invention aims to solve or at least mitigate the above and other problems.

Prior art documents include WO 2017/152125 A2 which discloses a chest drainage system which reduces or eliminates pooling of blood/liquid and/or clogging/clotting in the drainage tube and/or chest tube and provides objective and accurate measures of drained fluid volume and chest air leak. The chest drainage system continuously monitors chest tube and drainage tube status and clears pooled liquid in the drainage tube, and/or a clogged chest tube when necessary to restore negative pressure to the chest.

### SUMMARY

An object of the present invention is to provide a new type of drainage device which is improved over prior art, and which eliminates or at least mitigates the drawbacks discussed above. More specifically, an object of the invention is to provide a drainage device with a pressure relief valve that offers pain relief to patients in need of drainage. These objects are achieved by the technique set forth in the appended independent claims with preferred embodiments defined in the dependent claims related thereto.

According to embodiments of the invention, the above problems are solved by a pressure relief valve provided in the drainage device.

In a first aspect, a drainage device for removing fluid from a body cavity of a patient is provided. The device comprises a drainage tube, and a pressure sensing means. The drainage tube is connectable to a pump unit, which is configured to apply a suction pressure to the fluid in the body cavity, and the pressure sensing means is configured to detect when the applied suction pressure is at or above a predetermined threshold level. This is advantageous since the patient may experience the pain by a pressure exceeding the threshold level. By detecting the pressure, the pain can be relieved.

In one embodiment, the pressure sensing means is a pressure relief valve. The valve is configured to open when the pressure is at or above the predetermined threshold level. By opening the valve, the pain of the patient is relieved.

In another embodiment, the pressure relief valve, when open, is configured to allow air to flow through the drainage tube, thereby decreasing the suction pressure. A decreased suction pressure relieves the pain the patient experiences when the suction pressure becomes too high, i.e. exceeds a threshold level.

Preferably, the drainage device further comprises a non-return valve for preventing air from the pressure relief valve to enter the patient. It is advantageous from a patient security point of view not to let air travel in direction towards the patient. Complication of the patient may occur if air enters the patient.

The drainage device may further comprise a filter unit configured to remove pollutants from the air entering through the pressure relief valve. It is advantageous to remove possible pollutants from the air, which otherwise may accumulate inside the drainage device and cause a clog, a slower flow or reduced suction pressure.

The pressure sensing means comprises according to the invention a pressure sensor and a memory metal valve configured to control the suction pressure applied to the fluid in the body cavity. This is advantageous since a memory metal is a simple solution without need of additional sensors or electronics. Further, a memory metal provides a high level of recoverable plastic strain.

Preferably, the pressure sensor is configured to transmit an electric current to the valve, thus causing the valve to expand and form an opening configured to allow air to flow therethrough, thereby decreasing the suction pressure.

In one embodiment, the drainage device further comprises a pressure indicator configured to alert when the pressure is at or above the threshold level. This is helpful for the patient. If the patient feels that the suction pressure is beginning to be uncomfortably high, he/she can feel relieved by the indicator, which confirms that the pressure is actually on the high side, and that the pressure soon is going to be lowered. This can help the patient to endure any pain symptoms.

Preferably, the pressure indicator is one or more of a visual indicator, such as a lamp, a LED, a propeller means, a colour indication means or a visible air flow, or an audial indicator, such as a whistle means, or a bellow means. All forms of visual or audial indicators provide the patient with information regarding the suction pressure having reached a threshold level.

In one embodiment, a first end portion of the drainage tube is connectable to a patient side tube of a patient, and a second end portion of the drainage tube is connectable to a collection unit for collection of the removed fluid. It is advantageous to use a collection unit for gathering the removed fluid. A portable collection unit also means that the patient needs not be completely stationary when the drainage procedure is performed.

The pressure sensing means is preferably arranged between the patient side tube and the drainage tube. This is a favourable location since this is where the two tubes meet.

In one embodiment, the patient side tube is a chest tube. Fluid is often drained from the lung area of a patient, and in this case, the patient tube is a chest tube.

The predetermined suction pressure threshold level is preferably between 20-60 cmH₂O. This is a suitable threshold level for most patients.

The present invention is defined in claim 1.

### BRIEF DESCRIPTION OF THE DRAWINGS

Embodiments of the invention will be described in the following; references being made to the appended diagrammatical drawings which illustrate non-limiting examples of how the inventive concept can be reduced into practice.
Fig. 1 is a front view of a patient with pleural effusion;
Fig. 2a is a front view of a drainage device according to one embodiment;
Fig. 2b is a perspective view of a drainage tube and a collection means according to one embodiment;
Fig. 3a is a perspective view of a loop of the drainage tube in Fig. 2b and a first fixation means;
Fig. 3b is a perspective view of a second fixation means;
Fig. 3c is a perspective view of the loop of the drainage tube in Fig. 3a arranged around a peristaltic mechanism;
Fig. 4 is a perspective view of the drainage device according to one embodiment;
Fig. 5 is a detail view of the drainage device in Fig. 4;
Fig. 6 is a perspective view of the drainage tube and collection means in Fig. 2b, and a detail view of a pressure sensing means;
Fig. 7 is a side view of a part of the drainage device;
Fig. 8 is a s side cross section view of a pressure relief valve according to one embodiment in a closed position;
Fig. 9 is a side cross section view of the pressure relief valve in Fig. 8 in an open position;
Fig. 10 is a top view of the pressure relief valve in Figs 8-9
Fig. 11a is a top view of the pressure relief valve according to one embodiment in a closed position;

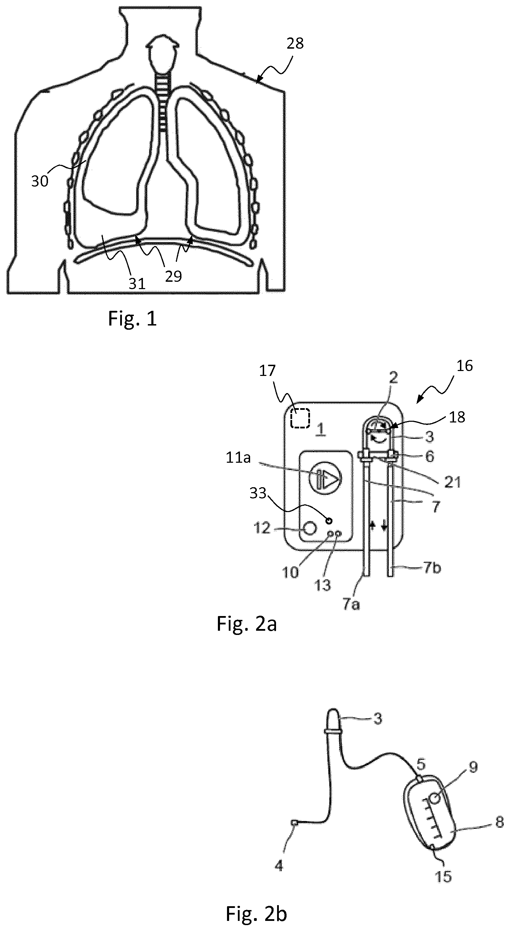

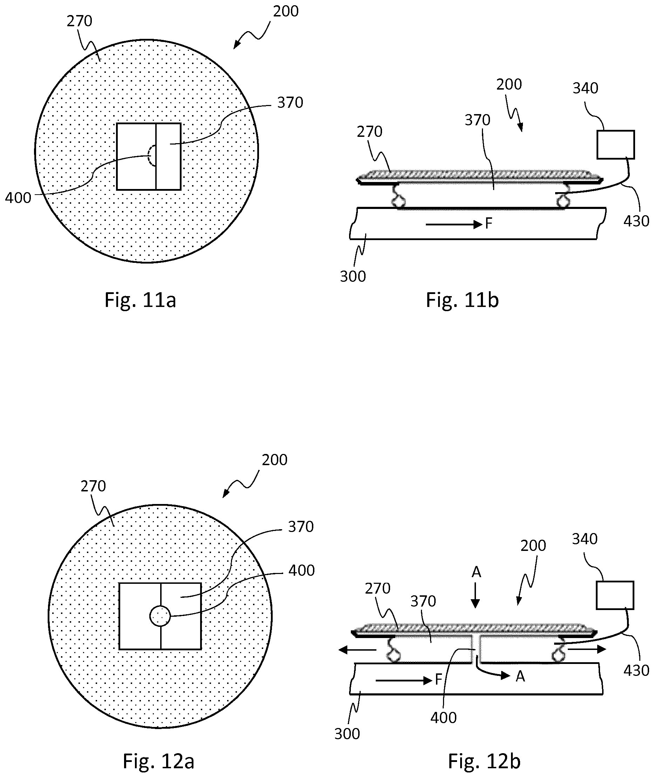

Fig. 1 1b is a side view of the pressure relief valve in Fig. 11a in the closed position;
Fig. 12a is a top view of the pressure relief valve in Figs 11 in an open position; and
Fig. 12a is a side view of the pressure relief valve in Figs 11-12a in the open position.

### DETAILED DESCRIPTION

Hereinafter, certain embodiments will be described more fully with reference to the accompanying drawings. The invention may, however, be embodied in many different forms and should not be construed as limited to the embodiments set forth herein; rather, these embodiments are provided by way of example so that this disclosure will be thorough and complete, and will fully convey the scope of the invention, such as it is defined in the appended claims, to those skilled in the art. If nothing else is stated, different embodiments may be combined with each other.

Fig. 1 shows a patient 28 with lungs 29. The lungs 29 are surrounded by lung lining, or pleura 30. When the patient 28 suffers from e.g. different forms of cancer diseases, fluid may accumulate between the lung 29 and the lung lining 30. This condition is referred to as pleural effusion 31. This condition is treated by drainage by means of a drainage device 16 as will be described below. As such device 16 is not monitored at all times by medical staff, a problem may occur when the excess fluid has been removed from the patient 28. The drainage device 16 continues to run until it is being shut down, and during this time the continued suction pressure causes the lung to become entrapped, resulting in severe pain to the patient 28.

Figs 2a and 2b show a front view of the drainage apparatus 16 and a perspective view of a part of the apparatus 16 respectively, according to one embodiment. The drainage apparatus is also referred to below as a drainage device, or a drainage system. The system/device 16 is disposable. That is, it is to be used once only, and thereafter be thrown away, such as to not spread infections between patients 28.

As seen in Fig. 2a, the drainage apparatus 16 comprises a housing 1 for housing major components of the apparatus 16, a, preferably flexible, drainage tube 3, and a peristaltic mechanism 2. The drainage tube 3 has a first end portion 7a, provided with a patient side connector 4 for connecting the device to an access port (not shown) of a patient 28, and a second end portion 7b, provided with a device side connector 5. A collection unit 8 is preferably provided, which is connectable to the device side connector 5 of the drainage tube 3.

The drainage apparatus 16 may further comprises a pressure regulator 17.

The depicted device further comprises at least one liquid property indicator 9, a peristaltic movement indicator 10, a peristaltic regulator 11, a peristaltic regulator button 11a, a power button 12, a battery power indicator 13. Further, a sample port 15 is provided at a bottom end of the collection unit 8 to enable samples from the drained fluid to be extracted for further testing.

The drainage tube 3 is inserted through the peristaltic mechanism 2 and is fixed in its operating position by first fixation means 6 that fixes the operating position of said drainage tube 3 and provides optimal operating peristaltic conditions.

When the drainage tube 3 and the two connectors 4, 5 are in position, the device may be activated by pushing the power button 12. Thus, the device is ready for use.

To use the drainage apparatus, a patient's access port is connected to the patient side connector 4.

Pressing the peristaltic regulator button 11 activates the drainage procedure and the peristaltic pumping is indicated by the peristaltic movement indicator 10. As the bodily fluid is drained from the bodily cavity it enters the first portion 7a of the drainage tube 7 via the patient side connector 4, passes the peristaltic mechanism 2 into the second end portion 7b of the drainage tube 3, and then enters the collection unit 8 via the device side connector 5.

The peristaltic regulator 11 controls a peristaltic motor in a preprogrammed manner to perform what may be denoted a "drainage cycle". To provide this preprogrammed manner of control the peristaltic regulator 11 is configured to include an acceleration phase during which the peristaltic motor is controlled to accelerate from an rpm of zero revolutions per minute up to a predetermined operational rpm during a first predetermined time period.

The peristaltic regulator 11 is further configured to subsequently regulate the motor to keep the predetermined operational rpm during a second predetermined time period.

The peristaltic regulator 11 is further configured to subsequently, during a third predetermined time period, decelerate the peristaltic motor from the operational rpm down to an rpm of zero revolutions per minute.

The first predetermined time period may preferably be chosen in the interval of 20-40 seconds, the second predetermined time period may preferably be chosen in the interval of 150 to 250 seconds, and the third predetermined time period may preferably be chosen in the interval of 20-40 seconds. Most preferably the predetermined time periods are chosen as around 30, 200, and 30 seconds respectively.

The peristaltic movement indicator 10 is configured to indicate to the user how much of the peristaltic movement capacity that has been utilized. The peristaltic mechanism is rotating and the regulator 11 controls the rotational speed. If the battery power supply reaches critical levels, the battery power indicator 13, alerts the user to recharge the battery.

As liquid is transported through the second portion 7b of the drainage tube 3 and discharged through the device side connector 5 into the collection unit 8, it will be in contact with the liquid property indicator 9, and liquid properties such as pH and lactate are measured, as well as the totally accumulated volume.

The liquid property indicator(s) 9 is provided to determine properties of the drained liquid. Liquid properties are determined e.g. by means of chemical indicator(s) provided at the inside of the, preferably transparent, collection unit 8.

The pH-value of the drained fluid could be measure for instance by means of a pH indicator in the form of a halo chromic chemical compound such that the acidity or basicity can be visually determined. Another option to determine the pH of the drained fluid is by means of electronic circuits configured to receive and process signals from pH sensors provided on an inner surface of the collection unit 8, where the pH sensors are printed.

As described above, the liquid property indicator 9 may determine the presence of lactate, reflecting metabolic stress. This may be accomplished by means of a test strip that contains the immobilized substrate, L-lactate, and be visually determined by its colour intensity, or by means of electronic circuits arranged to receive and process signals from lactate sensors provided on an inner surface of the collection unit 8, where the sensors are printed.

The body drainage system is preferably provided with a display unit (not shown), and the system is configured to display pH and/or lactate measurements on the display unit. The display unit may be arranged on, or as an integral part, of the drainage apparatus 16 or the housing 1, or may alternatively be arranged at a stand or hanger (not shown) for the collection unit 8.

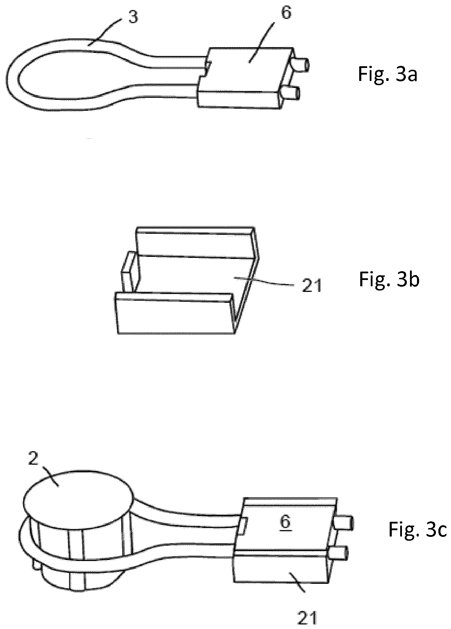

Fig. 3a shows a loop of the drainage tube 3 and the first fixation means 6. The tube 3 is firmly attached to the fixation means 6 which do not allow for any sliding between them. The first fixation means 6 is preferably provided with mechanical protrusions, indentations or other suitable means to enable the tube 3 forming the loop to be mounted in a certain position only.

The second fixation means 21, shown in Fig. 3b, is preferably also provided with mechanical protrusions, indentations, or other suitable means, inverted in relation to those of the first fixation means 6, in order to enable the first fixation means 6 and the loop to be mounted in a predetermined position only, and prevent mounting of the first fixation means 6 the wrong way in the second fixation means 21. It is important for patient safety that the fixation means 6, 21 are not mounted in the wrong way since this may cause fluid to be pumped into the patient 28 instead of out of the patient.

Fig. 3c shows the loop of the drainage tube 3 mounted around the peristaltic mechanism 2. It can also be seen how the first fixation means 6 fits together with the second fixation means 21 to keep the drainage tube 3 in position for efficient pumping. In the body drainage apparatus 16, the portion of the drainage tube 3 arranged around the peristaltic mechanism 2 has a length of tube corresponding to a length of arc of 80 to 190 degrees of a rotational revolution of the peristaltic mechanism 2, such that the rollers of the peristaltic pump 18 compress this portion of the tube 3 during its course of action. The portion of the tube 3 preferably comprises a length of tube corresponding to a length of arc of 80 to 140 degrees of a rotational revolution of the peristaltic mechanism, or more preferred, 80 to 100 degrees, or most preferred 85 to 95 degrees.

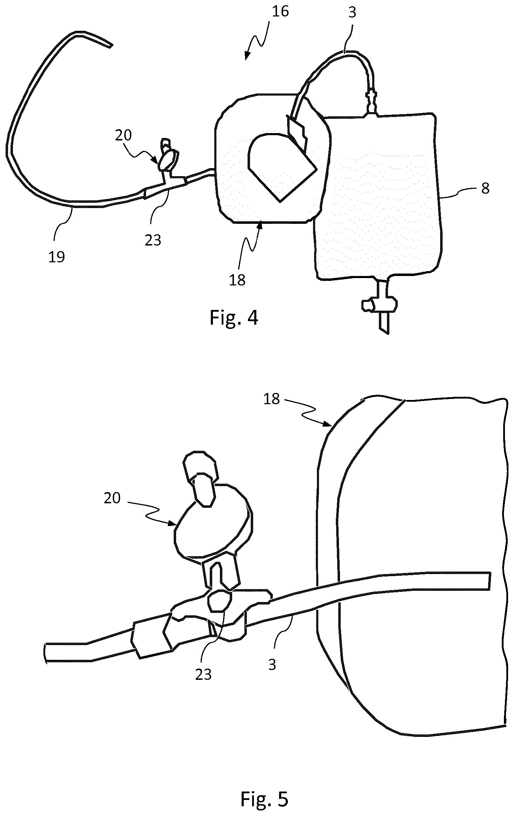

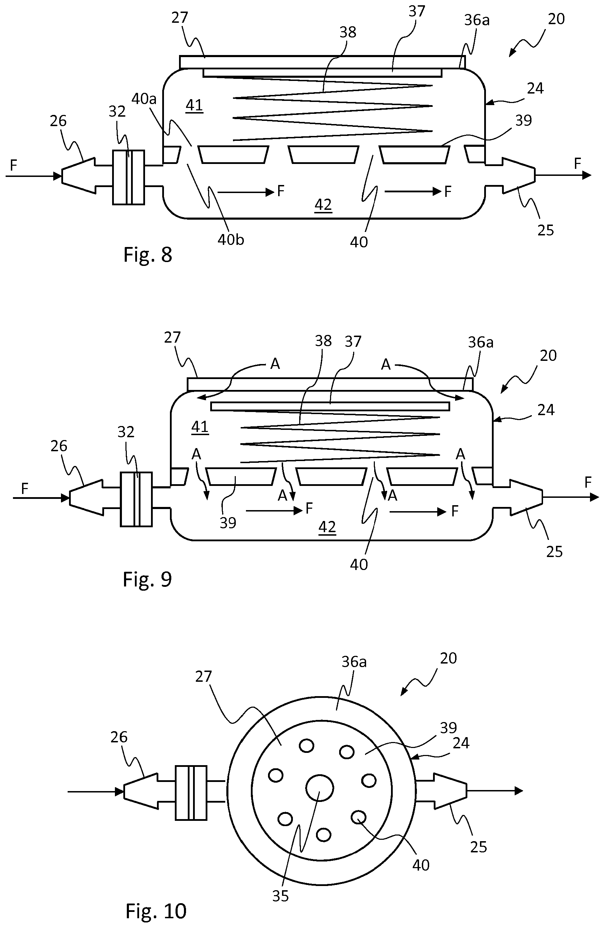

Figs 4-7 show the above described drainage apparatus 16 provided with a pressure relief valve arrangement 20, and figs 8-10 show the valve arrangement 20. The pressure relief valve 24 is configured to decrease the pressure applied by the drainage device 16 to a body cavity 31 of the patient 28. Thereby the discomfort and pain experienced by the patient 28 is relieved.

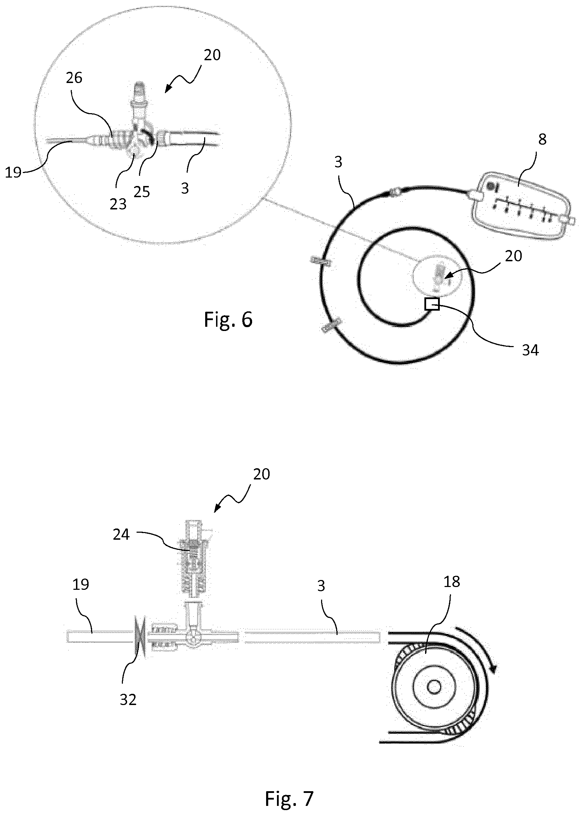

The pressure relief valve arrangement 20, which is best seen in Figs 6 and 8-10, comprises a pressure relief valve 24, a filter unit 27, a tap 23, a first connector 25 and a second 26 connector. The valve unit 24 further comprises a casing 36 from which the connectors 25, 26 protrude.

The filter unit 27 is arranged on a first surface 36a of the casing 36. The filter 27 is permeable to air such that ambient air may enter the interior of the valve casing 36, but impermeable to pollutions which are removed from the air entering the device 16.

Inside the casing 36, the valve 24 is provided with a valve membrane 37. The membrane 37 is connected to an actuator 38. The actuator is e.g. embodied as a spring, preferably as a coil spring. The interior of the casing 36 is further provided with a partitioning means 39, which preferably is embodied as a plate. The plate 39 divides the interior of the casing 39 into a first portion 41 and a second portion 42. The first portion 41 houses the valve membrane 37 and the spring 38. The second portion 42 is a flow channel for the drainage fluid.

The partitioning plate 39 is provided with through openings 40. The number of through openings 40 may vary between different embodiments and valve sizes. The in Fig. 10 shown number of seven openings 40 is to be seen as an example only. The entrance area 40a of the openings 40 is situated on the side of the partitioning means 39 facing the first portion 41 of the interior of the casing 36. The exit area 40b of the openings 40 is situated on the side of the partitioning means 39 facing the second portion 42 of the interior of the casing 36. The openings 40 are shaped as truncated cones. Thus, the entrance area 40a is larger compared to the exit area 40b. The openings 40 and their purpose will be further described below.

The pressure relief valve 24 is preferably a negative pressure relief valve. This means that the valve 24 opens when the pressure differential between the interior 41, 42 and the exterior of the drainage device 16 becomes too large, i.e. exceeds a certain threshold level. The first connector 25 is preferably a barbed tube fitting, and it is adapted to be connected to the drainage tube 3. The second connector 26 is adapted to be connected to a chest tube 19 of the patient 28.

Fluid which is drained from the patient 28 passes through the chest tube 19, entering the valve 24 via the second connector 26, travels through the flow channel 42 in the direction of arrows F, and exits the valve 24 via the first connector 25. This is shown in Fig. 8.

When the suction pressure of the drainage device 16 applied to a body cavity 31 of a patient 28 is at or above the predetermined threshold level, the pressure relief valve 24 opens, which is shown in Fig. 9. This is accomplished by the selection of the spring rate of the spring 38. When the suction pressure exceeds the spring rate of the spring 38 it compresses. The compressed spring 38 brings the valve membrane 37 towards the plate 39. Thus, a space is formed between the filter 27 and the membrane 37, providing an air flow A into the first portion 41 of the interior of the casing 36. The air flow A is led into the second portion 42 of the interior of the casing 36 by the openings 40. The air flow A is thus directed into the fluid flow channel 42, where it mixes with the fluid and continues into the drainage tube 3, via the first connector 25, thereby decreasing the suction pressure applied to the patient 28. The truncated cone-shape of the openings 40 prevent capillary forces on the fluid and thus prevents fluid from entering the first portion 41.

When/if the suction pressure normalizes, i.e. when the pressure drops to below the threshold pressure level the valve membrane 37 returns to its closed position. The air flow A is thus stopped.

The air A is forced in the direction of the collection unit 8 by the suction pressure exerted by the pump unit 18. Thus, the air A is prevented from entering the patient 28. For additional patient safety, the device 16 may be equipped with a non-return valve 32, which is preferably an in-line non-return valve. The air A remains in the collection unit 8 once it has entered therein.

The tap 23 is preferably a three-way tap. The tap 23 is used to turn off the pressure regulation. Even though the default setting is to use the pressure regulation, in some embodiments, it may be desired not to use it. Then, the tap 23 becomes useful.

In one embodiment, filter unit 27 is a micro pore filter. The filter acts as a valve in itself since a flow-resistance needs to be overcome before air can enter through it. As long as the resistance in the tube 19 is smaller compared to the resistance through the filter 24, no air will be let in. When the resistance through the tube 19 increases, air will begin to enter through the filter 27, i.e. through the valve, and into the device 16. Thus, the suction pressure acting on the patient 28 is decreased. In this embodiment, the actuator and the valve membrane are superfluous. However, the partitioning plate 39 is preferably provided such that the filter unit 27 is not in direct contact with the drained fluid. Thus, the filter 27 is arranged on a distance apart from the fluid, e.g. by means of the plate 39. If the filter 27 contacts the fluid, the flow resistance through the filter may become too large for air to pass therethrough, which means that the filter cannot open.

The drainage device 16 comprises a pressure indicator 35 which indicates when the threshold pressure has been reached. This may be a signal to the user 28 that sufficient drainage has been achieved. The pressure indicator 35 is e.g. a visual pressure indicator, or it is an audial pressure indicator, which alerts the user by means of sound. The visual pressure indicator may be in the form of a LED or a lamp, or alternatively, or additionally the visual pressure indicator 35 comprises colour indication means. The colour pressure indicator 35 is configured to change its color when it comes into contact with air. Thus, when the pressure relief valve 24 is opened and air is let through, the colour pressure indicator 35 change colour and the user is alerted.

Another visual option for the pressure indicator 35 is a propeller means. The propeller means 35 is configured to rotate during depressurization, i.e. when the pressure relief valve 24 is open. When the pressure is normalized, the propeller means 35 stands still. Thus, it becomes apparent to the user 28 when the valve is open. If the propeller means is made to exert a noise when it is rotating a combination of visible and audial indication is obtained.

Yet another visual pressure indicator 35 is the air flowing through the drainage tube 3 when the pressure relief valve 24 is open.

The audial pressure indicator 35 may comprise bellow means. The bellow means 35 has an expanded mode and a collapsed mode. When the pressure is too low, the bellow means 35 collapses and emit a sound. When the pressure is re-normalized, the bellow means 35 returns to its expanded mode.

An alternative audial pressure indicator 35 is a whistle means. When air is entering through the whistle means 35, i.e. when the pressure relief valve 24 is open, a sound is emitted. The whistle means may e.g. comprise a ball organ which produces a sound when air passes it.

In one embodiment, with reference to Figs 11a, 11b, 12a and 12b, the valve arrangement 200 comprises a filter unit 270 and a valve membrane 370. The pressure relief valve membrane 370 comprises a switch formed by a memory metal. The memory metal may also be referred to as one of SMA, smart metal, memory alloy, muscle wire, smart alloy. The valve membrane 370 of memory metal is arranged in operative connection with a pressure sensor 340.

When the suction pressure registered by the pressure sensor 340 is below the threshold pressure level, the valve 370 is closed, as is shown in Figs 11a and 11b. The fluid travels through pipe 300 in the direction of arrow F. No air is let through the valve 370.

With reference to Figs 12a-12b, when the pressure detected by the pressure sensor 340 exceeds the predetermined threshold value, an electric current 430 flows from the pressure sensor 340 to the valve 370. The current 430 heats the metal of the valve 370, causing the memory metal to expand. Thus, an opening 400 in the valve 370 is opened. Thus, air A can flow through the filter unit 270, through the opening 400 and into the fluid flow F, and thus relieving the suction pressure in the same way as described above in connection with Figs 8-10.

The connection between the valve arrangement 200 and the tube 300 is schematical only. Preferably, the connection comprises a connector valve of any suitable type. The valve arrangement 200 may be combined with features from the valve arrangement 20 as shown in Figs 8-10 and described in connection with these figures.

In one embodiment, the drainage device 16 comprises a micro controller 33, preferably arranged in the pump unit 18. The suction pressure applied by the drainage device 16 is controllable by means of the micro controller 33. The pressure regulation is conducted by means of for example an in-line pressure sensor 34 configured to communicate with the pump unit 18 via the micro controller 33.

In one embodiment, the pressure relief valve is substituted by a pressure sensor 34. When the pressure sensor 34 detects a pressure exceeding the predetermined threshold level, it transmits a signal to the pump unit 18, thereby disconnecting the pump unit 18. Alternatively, or additionally, the suction pressure applied by the pump unit 18 is regulated to a lower pressure.

A preferred pressure threshold level, at which the suction pressure is interrupted, is between 20-60 cmH₂O. One possibility is to set the suction pressure threshold level to 50-60 cmH₂O. This pressure level interval results in an efficient drainage. Another possibility is to use a lower threshold level. This is suitable for more sensitive patients 28. A cancer patient may experience a lot of pain and it is preferred not to expose him/her to unnecessary pain. Thus, in this case, a preferred threshold level is 20-40 cmH₂O. The predetermined threshold pressure could be pre-set, or it could be selectable from a variety of preset pressures, or it could be freely selectable, or it could be a combination of said options.

The foregoing description of embodiments of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of this invention as defined by the accompanying claims. For example, fluid may accumulate in other areas, apart from the lungs, of the body in connection with other medical conditions. The invention is thus not limited to drainage of the lung area.

## Claims

1. Drainage device for removing fluid from a body cavity of a patient, the device (16) comprising a drainage tube (3), and a pressure sensing means (24; 370), the drainage tube (3) being connectable to a pump unit (18), the pump unit (18) being configured to apply a suction pressure to the fluid in the body cavity (31), and the pressure sensing means (24; 370) being configured to detect when the applied suction pressure is at or above a predetermined threshold level,
**characterised in that** the pressure sensing means comprises a pressure sensor (340) and a memory metal valve (370) configured to control the suction pressure applied to the fluid in the body cavity (31).

2. The drainage device according to claim 1, wherein the pressure sensing means is a pressure relief valve (24), said valve (24) being configured to open when the pressure is at or above the predetermined threshold level.

3. The drainage device according to claim 2, wherein the pressure relief valve (24), when open, is configured to allow air (A) to flow through the drainage tube (3), thereby decreasing the suction pressure.

4. The drainage device according to any one of the claims 2-3, further comprising a non-return valve (32) for preventing air from the pressure relief valve (24) to enter the patient (28).

5. The drainage device according to any one of the claims 2-4, further comprising a filter unit (27) configured to remove pollutants from the air entering through the pressure relief valve (24).

6. The drainage device according to any of claims 1-5 , wherein the pressure sensor (340) is configured to transmit an electric current (430) to the valve (370), thus causing the valve (370) to expand and form an opening (400) configured to allow air (A) to flow therethrough, thereby decreasing the suction pressure.

7. The drainage device according to any one of the preceding claims, further comprising a pressure indicator (35) configured to alert when the pressure is at or above the threshold level.

8. The drainage device according to claim 7, wherein the pressure indicator (35) is one or more of a visual indicator, such as a lamp, a LED, a propeller means, a colour indication means or a visible air flow, or an audial indicator, such as a whistle means, or a bellow means.

9. The drainage device according to any one of the preceding claims,
wherein a first end portion (7a) of the drainage tube (3) is connectable to a patient side tube (19) of a patient (28), and a second end portion (7b) of the drainage tube (3) is connectable to a collection unit (8) for collection of the removed fluid.

10. The drainage device according to any one of the preceding claims,
wherein the pressure sensing means (24; 34) is arranged between the patient side tube (19) and the drainage tube (3).

11. The drainage device according to any one of the claims 9-10, wherein the patient side tube is a chest tube (19).

12. The drainage device according to any one of the preceding claims,
wherein the predetermined suction pressure threshold level is between 1961.28 Pascal and 5883.83 Pascal (20-60 cmH₂O).

13. The drainage device according to claim 1 for removing fluid from a body cavity of a patient, wherein the drainage device (16) further comprises a non-return valve (32) for preventing air from the pressure relief valve (24) to enter the patient (28).

## Patentansprüche

1. Drainagevorrichtung zum Entfernen von Fluid aus einer Körperhöhle eines Patienten, die Vorrichtung (16) umfassend einen Drainageschlauch (3) und ein Drucksensormittel (24; 370), wobei der Drainageschlauch (3) mit einer Pumpeneinheit (18) verbindbar ist, wobei die Pumpeneinheit (18) konfiguriert ist, um einen Saugdruck auf das Fluid in der Körperhöhle (31) auszuüben, und das Drucksensormittel (24; 370) konfiguriert ist, um zu erfassen, wenn der ausgeübte Saugdruck einen vorgegebenen Schwellenwert erreicht oder darüber ist,
**dadurch gekennzeichnet, dass** das Drucksensormittel einen Drucksensor (340) und ein Memory-Metall-Ventil (370) umfasst, das konfiguriert ist, um den Saugdruck, der auf das Fluid in der Körperhöhle (31) ausgeübt wird, zu steuern.

2. Drainagevorrichtung nach Anspruch 1, wobei das Drucksensormittel ein Druckablassventil (24) ist, wobei das Ventil (24) konfiguriert ist, um sich zu öffnen, wenn der Druck den vorgegebenen Schwellenwert erreicht oder darüber ist.

3. Drainagevorrichtung nach Anspruch 2, wobei das Druckablassventil (24), wenn geöffnet, konfiguriert ist, um zu ermöglichen, dass Luft (A) durch den Drainageschlauch (3) strömt, wobei dadurch der Saugdruck verringert wird.

4. Drainagevorrichtung nach einem der Ansprüche 2 bis 3, ferner umfassend eine Rückflusssperre (32) zum Verhindern, dass Luft aus dem Druckablassventil (24) in den Patienten (28) gelangt.

5. Drainagevorrichtung nach einem der Ansprüche 2 bis 4, ferner umfassend eine Filtereinheit (27), die konfiguriert ist, um Schadstoffe aus der Luft, die durch das Druckablassventil (24) gelangt, zu entfernen.

6. Drainagevorrichtung nach einem der Ansprüche 1 bis 5, wobei der Drucksensor (340) konfiguriert ist, um einen elektrischen Strom (430) an das Ventil (370) zu übertragen, wodurch bewirkt wird, dass das Ventil (370) sich vergrößert und eine Öffnung (400) ausbildet, die konfiguriert ist, um zu ermöglichen, dass Luft (A) dahindurch strömt, wobei dadurch der Saugdruck verringert wird.

7. Drainagevorrichtung nach einem der vorstehenden Ansprüche, ferner umfassend einen Druckindikator (35), der konfiguriert ist, um Alarm zu schlagen, wenn der Druck den Schwellenwert erreicht oder darüber ist.

8. Drainagevorrichtung nach Anspruch 7, wobei der Druckindikator (35) einer oder mehrere eines visuellen Indikators, wie etwa einer Lampe, einer LED, eines Propellermittels, eines Farbindikatormittels oder eines sichtbaren Luftstroms, und/oder eines akustischen Indikators, wie etwa ein Pfeif- oder Blasebalgmittel, ist.

9. Drainagevorrichtung nach einem der vorstehenden Ansprüche, wobei ein erster Endabschnitt (7a) des Drainageschlauchs (3) mit einem patientenseitigen Schlauch (19) eines Patienten (28) verbindbar ist und ein zweiter Endabschnitt (7b) des Drainageschlauchs (3) mit einer Auffangeinheit (8) für Auffangen des entfernten Fluids verbindbar ist.

10. Drainagevorrichtung nach einem der vorstehenden Ansprüche, wobei das Drucksensormittel (24; 34) zwischen dem patientenseitigen Schlauch (19) und dem Drainageschlauch (3) angeordnet ist.

11. Drainagevorrichtung nach einem der Ansprüche 9 bis 10, wobei der patientenseitige Schlauch ein Thoraxdrainageschlauch (19) ist.

12. Drainagevorrichtung nach einem der vorstehenden Ansprüche, wobei der vorgegebene Saugdruckschwellenwert zwischen 1961,28 Pascal und 5883,83 Pascal (20-60 cmH₂O) liegt.

13. Drainagevorrichtung nach Anspruch 1 zum Entfernen von Fluid aus einer Körperhöhle eines Patienten, wobei die Drainagevorrichtung (16) ferner eine Rückflusssperre (32) zum Verhindern, dass Luft aus dem Druckablassventil (24) in den Patienten (28) gelangt, umfasst.

## Revendications

1. Dispositif de drainage destiné à éliminer le liquide d'une cavité corporelle d'un patient, le dispositif (16) comprenant un tube de drainage (3), et un moyen de détection de pression (24 ; 370), le tube de drainage (3) pouvant être raccordé à une unité de pompage (18), l'unité de pompage (18) étant configurée pour appliquer une pression d'aspiration au fluide dans la cavité corporelle (31), et le moyen de détection de pression (24 ; 370) étant configuré pour détecter si la pression d'aspiration appliquée est égale ou supérieure à un seuil prédéterminé,
**caractérisé en ce que** le moyen de détection de pression comprend un capteur de pression (340) et une valve de métal à mémoire (370) configurée pour commander la pression d'aspiration appliquée au fluide dans la cavité de corps (31).

2. Dispositif de drainage selon la revendication 1, dans lequel le moyen de détection de pression est une valve de décompression (24), ladite valve (24) étant conçue pour s'ouvrir lorsque la pression est égale ou supérieure au niveau de seuil prédéterminé.

3. Dispositif de drainage selon la revendication 2, dans lequel la valve de décompression (24), lorsqu'elle est ouverte, est conçue pour permettre à l'air (A) de s'écouler dans le tube de drainage (3), diminuant ainsi la pression d'aspiration.

4. Dispositif de drainage selon l'une quelconque des revendications 2 et 3, comprenant en outre une valve anti-retour (32) pour empêcher l'air provenant de la valve de décompression (24) de pénétrer dans le patient (28).

5. Dispositif de drainage selon l'une quelconque des revendications 2 à 4, comprenant en outre une unité de filtrage (27) configurée pour éliminer les polluants de l'air pénétrant par la valve de décompression (24).

6. Dispositif de drainage selon l'une quelconque des revendications 1 à 5, dans lequel le capteur de pression (340) est configuré pour transmettre un courant électrique (430) à la valve (370), provoquant ainsi l'expansion de la valve (370) et la formation d'une ouverture (400) conçue pour permettre à l'air (A) de s'écouler dans celle-ci, diminuant ainsi la pression d'aspiration.

7. Dispositif de drainage selon l'une quelconque des revendications précédentes, comprenant en outre un indicateur de pression (35) configuré pour alerter lorsque la pression est égale ou supérieure au niveau seuil.

8. Dispositif de drainage selon la revendication 7, dans lequel l'indicateur de pression (35) est un ou plusieurs indicateurs visuels, tels qu'une lampe, une diode électroluminescente, une hélice, un indicateur de couleur ou un flux d'air visible, ou un indicateur sonore, tel qu'un moyen de sifflet ou un moyen de soufflet.

9. Dispositif de drainage selon l'une quelconque des revendications précédentes, dans lequel une première partie d'extrémité (7a) du tube de drainage (3) peut être raccordée à un tube latéral (19) d'un patient (28), et une seconde partie d'extrémité (7b) du tube de drainage (3) peut être raccordée à une unité de collecte (8) pour la collecte du fluide éliminé.

10. Dispositif de drainage selon l'une quelconque des revendications précédentes, dans lequel le moyen de détection de pression (24 ; 34) est agencé entre le tube côté patient (19) et le tube de drainage (3).

11. Dispositif de drainage selon l'une quelconque des revendications 9 à 10, dans lequel le tube côté patient est un tube thoracique (19).

12. Dispositif de drainage selon l'une quelconque des revendications précédentes, dans lequel le niveau seuil de pression d'aspiration prédéterminé est compris entre 1961,28 Pascal et 5883,83 Pascal (20 à 60 cmH₂O).

13. Dispositif de drainage selon la revendication 1, destiné à éliminer le fluide d'une cavité corporelle d'un patient, dans lequel le dispositif de drainage (16) comprend en outre une valve anti-retour (32) pour empêcher l'air de la valve de décompression (24) de pénétrer dans le patient (28).
